(19) Europäisches Patentamt
European Patent Office
Office européen des brevets

(11) EP 1 346 223 B1

# (12) EUROPÄISCHE PATENTSCHRIFT

(45) Veröffentlichungstag und Bekanntmachung des Hinweises auf die Patenterteilung:
**24.01.2007 Patentblatt 2007/04**

(21) Anmeldenummer: **01986432.1**

(22) Anmeldetag: **21.12.2001**

(51) Int Cl.:
***G01N 33/58*** *(2006.01)*

(86) Internationale Anmeldenummer:
**PCT/EP2001/015185**

(87) Internationale Veröffentlichungsnummer:
**WO 2002/054045 (11.07.2002 Gazette 2002/28)**

## (54) REAGENS ZUR LUMINESZENZ-OPTISCHEN BESTIMMUNG EINES ANALYTEN

REAGENT FOR LUMINESCENCE OPTICAL DETERMINATION OF AN ANALYTE

REACTIF POUR LA DETERMINATION OPTIQUE DE LUMINESCENCE D'UN ANALYTE

(84) Benannte Vertragsstaaten:
**AT BE CH CY DE DK ES FI FR GB GR IE IT LI LU MC NL PT SE TR**

(30) Priorität: **29.12.2000 AT 21612000**

(43) Veröffentlichungstag der Anmeldung:
**24.09.2003 Patentblatt 2003/39**

(73) Patentinhaber:
- **F.HOFFMANN-LA ROCHE AG**
  **4070 Basel (CH)**
- **Roche Diagnostics GmbH**
  **68305 Mannheim (DE)**
  Benannte Vertragsstaaten:
  **DE**

(72) Erfinder: **KLIMANT, Ingo**
**93098 Mintraching (DE)**

(74) Vertreter: **Schwarz, Albin et al**
**Wipplingerstrasse 32**
**1010 Wien (AT)**

(56) Entgegenhaltungen:

| | |
|---|---|
| **EP-A- 0 381 026** | **WO-A-00/42438** |
| **WO-A-02/054076** | **US-A- 5 194 393** |
| **US-A- 5 585 235** | **US-A- 5 952 491** |

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein lumineszenz-optisches Verfahren zur qualitativen und quantitativen, Bestimmung zumindest eines Analyten bzw. einer Komponente eines flüssigen Meßmediums, welche einen auf die zu bestimmende Komponente direkt oder indirekt durch Änderung seines Absorptionsspektrums reagierenden Chromophor (oder Luminophor) und einen auf die zu bestimmende Komponente nicht ansprechenden Luminophor aufweist, wobei das Emissionsspektrum des Luminophors zumindest teilweise mit dem Absorptionsspektrum des Chromophors überlappt und der strahlungslose Energietransfer zwischen Luminophor und Chromophor zumindest eine Lumineszenzeigenschaft des Luminophors meßbar ändert. Dieses Prinzip ist als sogenanntes FRET-Prinzip bekannt

Die Erfindung betrifft weiters ein Reagens zur quantitativen Bestimmung zumindest eines Analyten bzw. einer Komponente eines gasförmigen oder flüssigen Meßmediums, welche einen auf die zu bestimmende Komponente direkt oder indirekt durch Änderung seines Absorptionsspektrums reagierenden Chromophor (oder Luminophor) und einen auf die zu bestimmende Komponente nicht ansprechenden Luminophor aufweist, wobei das Emissionsspektrum des Luminophors zumindest teilweise mit dem Absorptionsspektrum des Chromophors überlappt und der strahlungslose Energietransfer zwischen Luminophor und Chromophor zumindest eine Lumineszenzeigenschaft des Luminophors meßbar ändert.

Im folgenden werden unter Luminophore Farbstoffe verstanden, welche nach entsprechender Strahlungsanregung Phosphoreszenz- oder Fluoreszenzstrahlung emittieren. Das Absorptionsspektrum des Chromophors wird entweder direkt durch die zu messende Komponente oder indirekt durch ein chemisches Reaktionsprodukt der zu messenden Komponente beeinflußt. Unter "quantitative Bestimmungen einer chemischen Komponente" wird sowohl die Bestimmung der Konzentration, der Aktivität als auch des Qaspartialdruckes verstanden, wobei aus den Werten von zumindest einer Lumineszenzeigenschaft des Luminophors auf die Meßgröße geschlossen wird.

Ein Verfahren und ein Sensor, bei welchem pH- und kationensensitive Chromophore (Akzeptor) vorzugsweise kovalent an einen Luminophor (Donor) gebunden sind, sind aus der US 5,232,858 bekannt. Aus der Lumineszenzabklingzeit des Luminophors wird auf den pH-Wert bzw. auf die Konzentration des zu bestimmenden Kations im Meßmedium geschlossen.

Zum Stand der Technik ist ferner die US 5,648,269 zu nennen. Dieses Dokument schlägt die Verwendung der scheinbaren Lumineszenzabklingzeit des Luminophors zur Bestimmung der Meßgröße vor. Bei Luminophoren mit einer Abklingzeitkomponente ist die scheinbare Lumineszenzabklingzeit identisch der effektiven Abklingzeit. Bei Luminophoren mit mehreren Abklingzeitkomponenten ist die scheinbare Abklingzeit einfacher zu erheben, hat jedoch den Nachteil, daß - insbesondere bei nicht robusten Systemen - die Fehler größer werden.

Lumineszenzabklingzeiten können durch phasen-modulations bzw. zeitaufgelöste Lumineszenzmeßtechniken erhalten werden.

Ein ähnliches Verfahren ist aus der EP-A - 0 214 768 bekannt. Hier wird aus der gemessenen Lumineszenzintensität auf die Konzentration des zu bestimmenden Parameters im Meßmedium geschlossen.

Die strahlungslose Energietransferrate von Donor- zu Akzeptormolekülen hängt von der räumlichen Nähe der Moleküle beider Substanzen ab. Die Transferrate $k_T(r)$ ist extrem empfindlich vom räumlichen Abstand r zwischen Donor und Akzeptor und klingt mit der 6ten Potenz des Abstandes ab

$$k_T(r) = \frac{1}{\tau_D}\left(\frac{R_o}{r}\right)^6,$$

wobei $\tau_D$ die Lumineszenzabklingzeit des Donors in Abwesenheit des Akzeptors und Ro die charakteristische Försterdistanz bedeuten. Letzteres ist jene Donor-Akzeptor Distanz bei welcher eine 50%tige Effizienz des Energietransfers vorliegt. Je nach Donor-Akzeptor Paar liegt Ro zwischen 2 und 9 Nanometer.

Infolge des strahlungslosen Energietransfers von Donor- zu Akzeptormolekülen ändern sich die makroskopisch bestimmbaren Werte der lumineszenzoptischen Parameter (Lumineszenzquantenausbeute, Lumineszenzintensität, Lumineszenzabklingzeit) des Luminophors besonders effizient, wenn eine substantielle Anzahl von Molekülen beider Substanzen in engen räumlichen Kontakt gebracht wird.

In der US 5,194,393 A wird ein Sensor zur Durchführung immunoanalytischer Messungen beschrieben, bei welchem mit einem Farbstoffmolekül markierte Bindungskomponenten mit weiteren Bindungskomponenten reagieren, die an eine mit einem weiteren Farbstoffrnolekül versehene Langmuir-Blodgett-Schicht gebunden sind, welche ihrerseits auf einem festen Träger aufgebracht ist.

Die US 5,585,235 A beschreibt ein Verfahren zur Messung der Aktivität des Cholesterinestertransferproteins in einer Probe. Dabei werden Emulsionspartiket, die in ihrem Kern fluoreszenzmarkierte neutrale Lipide enthalten, zur

Probe hinzugefügt, wobei deren Emissionswellenlängenverschiebung ein Maß für den Einfluss störender Komponenten darstellt.

Zur Herstellung des engen räumlichen Kontaktes wird in der US 5,232,858 die kovalente Verbindung von Donor- und Akzeptormolekülen vorgeschlagen. In der EP-A - 0 214 768 werden individuelle Donor- und Akzeptormoleküle kovalent an die Oberfläche eines gemeinsamen Substrates, z. B. Glas, gebunden.

Die in der US 5,232,858 beschriebene kovalente Verknüpfung von Donor- und Akzeptormolekülen hat den Vorteil, daß der mittlere räumliche Abstand von Donor/Akzeptor möglichst konstant gehalten werden kann. Nachteilig ist jedoch, daß der synthetische Aufwand zur Herstellung kovalenter Verbindungen vorteilhafter Luminophore mit geeigneten pH- oder ionensensitiver Chromophoren besonders hoch ist.

Die kovalente Anbindung von Donor- und Akzeptormolekülen an die Oberfläche eines gemeinsamen Substrates (EP-A - 0 214 768) ist ebenfalls sehr aufwendig und hat vor allem den Nachteil, daß Grenzflächeneffekte die Qualität der Meßergebnisse nachteilig beeinflussen.

In der US 5,942,189 und in der US 6,046,055 wird daher vorgeschlagen, daß der Luminophor und der Chromophor elektrisch unterschiedlich geladene ionische Substanzen sind, welche in Form von Ionenpaaren in einem für die zu bestimmende chemische Komponente permeablen Matrixmaterial vorliegen.

Für die breite kommerzielle Anwendung besonders wichtig ist die Verwendung langlebiger Luminophore (mit Lumineszenzabklingzeiten > 100ns, vorzugsweise > 1 $\mu$s), wie sie beispielsweise Metall-Ligand-Komplexe, bestimmte Porphyrine und Lanthanide aufweisen. Bei Vorliegen langlebiger Lumineszenz können die opto-elektronischen Anordnungen und Komponenten zur Bestimmung der Lumineszenzabklingzeit bzw. daraus ableitbare Größen (z.B. mittlere Lumineszenzabklingzeit, Phasenwinkel) mittels Phasen- oder zeitaufgelöster Methoden besonders kostengünstig bestimmt werden.

Der Erfinder der vorliegenden Anmeldung hat jedoch erkannt, daß die oben beschriebenen, vorbekannten Verfahren den gemeinsamen Nachteil haben, daß insbesondere die Lumineszenz langlebiger Luminophore durch eine Reihe von Komponenten des Meßmediums beeinflußt wird. Eine bekannte Eigenschaft derartiger Luminophore ist die besonders große Abhängigkeit der Lumineszenzeigenschaft vom O2 Gehalt der Probe. Konsequenterweise werden solche Luminophore daher häufig zur Bestimmung des O2-Gehaltes eingesetzt (EP-A - 0 907 074). Bei Verwendung dieser Luminophore als Donorfarbstoffe bei auf dem FRET-Prinzip basierenden Sensoren ist es daher erforderlich, den O2-Gehalt des Meßmediums genau zu kennen oder zu bestimmen und entsprechende Korrekturen durchzuführen. Beispiele für weitere, die Lumineszenzquantenausbeute beeinflussende Substanzen sind Amine und Wasser. Bei kontinuierlichen Messungen (Monitoring) können Lumineszenzfarbstoffe durch den entstehenden Singulett-02 ganz oder teilweise zerstört werden. Entsprechend wurden Additive vorgeschlagen, welche diesen Prozess eingrenzen sollen. Ein genereller Nachteil bekannter, vorteilhafter Lumineszenzfarbstoffe ist jedoch die Empfindlichkeit der Lumineszenzeigenschaften gegenüber geringfügigen Veränderungen der chemischphysikalischen Mikroumgebung, hervorgerufen durch beliebige Komponenten der Probe, insbesondere Wasser. Dies führt bei Probenmedien unbekannter bzw. variabler chemischer bzw. biochemischer Zusammensetzung zu einer signifikanten Einschränkung der Meßgenauigkeit. Beispielsweise werden in der medizinischen Diagnostik im Bereich der Bestimmung des Blut-pH Reproduzierbarkeiten von +/- 5 milli-pH erwartet.

Aufgabe der Erfindung ist es, auf dem FRET-Prinzip beruhende lumineszenzoptische Bestimmungsmethoden, basierend auf einem Luminophor (Donor) und einem Chromophor (Akzeptor, Indikator), welcher die zu bestimmende Substanz (Analyt) oder deren Reaktionsprodukte reversibel bindet, hinsichtlich der Störanfälligkeit durch Komponenten der zu messenden Probe zu verbessern. Ferner sollen besonders aufwendige chemische Syntheseschritte zur Realisierung der räumlichen Nähe einer substantiellen Anzahl von Akzeptor- und abgeschirmten Donormolekülen vermieden werden.

Diese Aufgabe wird erfindungsgemäß dadurch gelöst, daß der Luminophor (Donor) und der Chromophor (Akzeptor) sich in unterschiedlichen chemischen Phasen befinden. Das Matrixmaterial der Donorphase soll für die Lumineszenzeigenschaften des Donors beinflussende Komponenten des Probenmediums impermeabel sein. Das Matrixmaterial der Akzeptorphase soll für den Analyten bzw. dessen Reaktionsprodukte permeabel sein.

Die Erfindung betrifft somit ein Reagens zur Durchführung von Analysen nach dem FRET-Prinzip arbeitet, enthaltend einen Akzeptorfarbstoff sowie Partikel, die einen Donorfarbstoff aufweisen, wobei sich Donorfarbstoff und Akzeptorfarbstoff in verschiedenen chemischen Phasen befinden, der Akzeptorfarbstoff an die Partikel gebunden vorliegt und die Partikel für das Probenmedium oder für die Lumineszenzeigenschaften des Donorfarbstoffes beeinflussende Komponenten des Probenmediums impermeabel sind.

Das Matrixmaterial der Donorphase liegt somit in Form kleiner Partikel dispergiert in der zu analysierenden Substanz vor, wobei die Akzeptormoleküle an der Oberfläche der Partikel gebunden sind.

Dabei kann der Chromophor adsorptiv oder elektrostatisch an der Oberfläche gebunden sein, oder, besonders bevorzugt, kovalent an funktionelle Gruppen gebunden sein.

In einer bevorzugten Ausführungsform werden als Materialien für die den Donorfarbstoff enthaltenden Partikel nicht weichgemachte Polymere, z.B. Polyacrylnitril und Derivate davon, PVC und/oder Polyvinylidenchlorid, eingesetzt.

Das erfindungsgemäße Verfahren zur qualitativen und/oder quantitativen Bestimmung zumindest eines Analyten bzw. einer Komponente eines flüssigen Meßmediums umfaßt die Zugabe des erfindungsgemäßen Reagens zum Meßmedium.

Es müssen somit lediglich die den Donorfarbstoff enthaltenden Partikel mit dem an die Partikel gebundenen Akzeptorfarbstoff dem Meßmedium, z.B. in einer Meßküvette zugegeben werden, wonach in an sich bekannter Weise die jeweilige Analyse durchgeführt wird. Das Meßmedium ist somit zugleich die Akzeptorphase.

Das erfindungsgemäße Verfahren wird bevorzugt zur Bestimmung des pH-Wertes einer Probe oder zur Bestimmung der Konzentration bzw. der Aktivität von Ionen, insbesondere von $Li^+$, $Na^+$, $K^+$, $Mg^{++}$, $Ca^{++}$ oder $Cl^-$ in einer Probe eingesetzt.

Weitere Anwendungsgebiete des erfindungsgemäßen Verfahrens betreffen sogenannte Transducer. Hierbei reagiert der Chromophor nicht direkt mit dem Analyten, sondern indirekt. Beispiele hierfür sind sogenannten enzymatische Sensoren (bzw. zur Bestimmung von Harnstoff und Kreatinin). Hierbei reagieren ein oder mehrere Enzyme mit dem Analyten, wobei ein Produkt entsteht, welches direkt mit dem Chromophor reagiert.

Bevorzugt ist das Meßmedium eine Körperflüssigkeit, insbesondere Blut, Plasma oder Serum.

Besonders vorteilhafte, erfindungsgemäß eingesetzte Luminophore (Donor) sind solche, die sich durch eine hohe Lumineszenzquantenausbeute und eine lange Lumineszenzabklingzeit ( > 100 ns) auszeichnen. Bevorzugte Luminophore sind kationische metallorganische Komplexe des Palladiums, Rhodiums, Platins, Rutheniums, Osmiums, der seltenen Erden (insbesonders Europium und Lanthan). Der organische Teil dieser metallorganischen Komplexe kann z.B. aus Liganden aus der Gruppe der Porphyrine, Bipyridyle, Phenanthroline oder anderer heterozyklischer Verbindungen bestehen.

Bevorzugte pH- und kationensensitive Chromophore (Akzeptor) sind anionische Substanzen, deren Lichtabsorption sich durch direkte oder indirekte chemisch/physikalische Wechselwirkung mit der zu bestimmenden Komponente des Probenmediums verändert, und deren Absorptionsspektrum sich zumindest teilweise mit dem Emissionsspektrum des Luminophors überlappt.

**I**) Bestimmung des pH-Wertes einer Probe

Optische Sensoren zur Bestimmung des pH-Wertes enthalten gemäß Stand der Technik (M.J.P. Leiner and O.S. Wolfbeis "Fiber Optic pH Sensors" in O.S. Wolfbeis "Fiber Optic Chemical Sensors and Biosensors", CRC-Press, Boca Raton, 1991, Vol. I, Chapter 8) zumeist einen in einer ionenpermeablen, vorzugsweise hydrophilen Polymermatrix vorliegenden Absorptionsfarbstoff (Chromophor) oder Fluoreszenzfarbstoff. In Abhängigkeit des pH-Wertes (pH = -log (aH+)) des Probenmediums stellt sich ein thermodynamisches Gleichgewicht zwischen den protonierten und deprotonierten Formen des Chromophors bzw. Fluorophors ein. Aus dem mit optischen Methoden meßbaren Konzentrationsverhältnis beider Formen kann auf den pH-Wert des Probenmediums rückgeschlossen werden.

Reaktion:

$$AH \xleftrightarrow{K_d} A^- + H^+$$

Gleichgewicht

$$K_d = \frac{cA^- * cH^+}{cAH}$$

.

AH ist die protonierte und $A^-$ ist die deprotonierte Form des pH sensitiven Chromophors. $H^+$ steht für ein Proton. $K_d$ ist die Gleichgewichtskonstante. c steht für Konzentration.

In der eingangs erwähnten US 5,232,858 werden pH-sensitive Chromophore beschrieben, welche vorzugsweise kovalent an einen pH-insensitiven Luminophor (Donor) gebunden werden. Aus der Lumineszenzabklingzeit des Luminophors (L) wird auf den pH-Wert der Meßlösung geschlossen.

Zur erfindungsgemäßen lumineszenzoptischen pH-Bestimmung wird beispielsweise als Akzeptorfarbstoff ein pH-sensitiver Chromophor verwendet, welcher in der Akzeptorphase vorliegt bzw. zumindest in direktem Kontakt zur Akzeptorphase steht, wobei die Akzeptorphase das zu analysierende Medium (Meßmedium) ist.

Bei niedrigen pH-Werten (pH << pK des Chromophors) des Probenmediums liegt der Chromophor vollständig in der protonierten Form vor. Wegen der minimalen spektralen Überlappung der Absorptionsbande des deprotonierten Chromophors und der Emissionsbande des Luminophors erreicht die strahlungslose Energietransferrate vom Luminophor zum Chromophor ein Minimum. Entsprechend erreichen die Werte der mittleren Lumineszenzabklingzeit und der relativen Lumineszenzintensität des Luminophors ein Maximum.

Bei hohen pH-Werten (pH >> pKa des Chromophors) des Probenmediums liegt der Chromophor vollständig in der deprotonierten Form vor. Wegen der maximalen spektralen Überlappung der Absorptionsbande des deprotonierten Chromophors und der Emissionsbande des Luminophors erreicht die strahlungslose Energietransferrate vom Luminophor (Donor, Donorfarbstoff) zum Chromophor (Akzeptor, Akzeptorfarbstoff) ein Maximum. Entsprechend erreichen die Werte der mittleren Lumineszenzabklingzeit und der relativen Lumineszenzintensität des Luminophors ein Minimum.

Bei pH-Werten des Probenmediums im Bereich von +/- 1,5 pH-Einheiten vom pKd-Wert (pKd = -log Kd) des Chromophors, kann aus der mittleren Lumineszenzabklingzeit oder der relativen Lumineszenzintensität des Luminophors mit ausreichender Genauigkeit auf den pH-Wert des Probenmediums geschlossen werden.

**II**) Bestimmung der Konzentration bzw. der Aktivität von Kationen und Anionen in einer Probe (Li+, Na+, K+, Mg++, Ca++, Cl-)

Bisher bekannt gewordene optische Sensoren bzw. optische Meßverfahren zur Bestimmung der Konzentration bzw. Aktivität von Kationen in einem Probenmedium beruhen auf unterschiedlichen Verfahren. So beschreibt die eingangs erwähnte erwähnten US 5,232,858 kationensensitive Chromophore (Chromoionophore), welche vorzugsweise kovalent an einen kationeninsensitiven Luminophor gebunden werden.

Bei sehr hohen Kationenkonzentrationen ($cY^{+p}$ >> Kd des Chromophors) des Probenmediums liegt der Chromophor vollständig in der komplexierten Form vor. (Y ist das zu bestimmende Kation, +p ist seine Ladungszahl). Bei sehr niedrigen Kationenkonzentrationen ($cY^{+p}$ << Kd des Chromophors) des Probenmediums liegt der Chromophor in freier, unkomplexierten Form vor.

Liegt die logarithmische Konzentration $\log(cY^{+p})$ des zu bestimmenden Kations des Probenmediums im Bereich von -log(Kd) +/-1.5, so kann mit ausreichender Genauigkeit aus der mittleren Lumineszenzabklingzeit bzw. der relativen Lumineszenzintensität des Luminophors auf die Konzentration des zu bestimmenden Kations im Probenmedium geschlossen werden.

Erfindungsgemäß eingesetzte pH-sensitive Chromophore sind in der nachfolgenden Tabelle 1 beispielhaft angeführt.

Tabelle 1: pH-sensitive Chromophore

| Chromophor | Absorptionswellenlänge [nm] protoniert/deprotoniert | pKa |
|---|---|---|
| Triphenylmethanfarbstoffe: | | |
| Bromphenolblau | 430/617 | 3.8 |
| Bromthymolblau | 430-435 / 615-618 | 6.7 |
| Dibromoxylenolblau | 420/614 | 7.6 |
| Azofarbstoffe: | | |
| Calmagit | 530/605 | 8.0 |
| Nitrazingelb | 460/590 | 6.5 |
| Sonstige: | | |
| o-Chlorophenol-indophenol | 555/625 | 7.1 |
| Naphthol-phthalein | 428/661 | 6.7 ,7.9 |

Weiters finden pH-sensitive Triphenylmethanfarbstoffe der allgemeinen Form

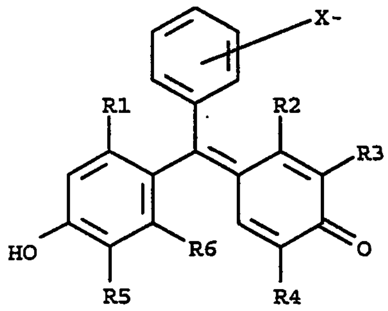

Verwendung. R1-6 können unabhängig voneinander H, Halogenatome, Nitrogruppen und Alkylgruppen sein. $X^-$ steht für eine optionelle Gruppe zur kovalenten Immobilisierung des Chromophors. Geeignete Gruppen sind beispielsweise $-(CH_2)n-SO3^-$ oder $-(CH2)n-COO^-$, - (CH2)n-NH2 (n=0-18)
pH sensitive Azofarbstoffe der allgemeinen Form,

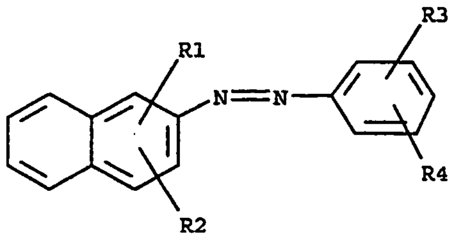

wobei R1-R4 unabhängig voneinander Substituenten, beispielsweise Halogenatome, Nitrogruppen bzw. Alkylgruppen und für die kovalente Immobilisierung geeignete Gruppen sind, - aber mindestens eine -OH Gruppe vertreten sein muß.

Erfindungsgemäß anwendbare kationensensitive Chromophore:

Erfindungsgemäß anwendbare, kationensensitive Chromoionophore zur Bestimmung von Lithium-, Kalium-, Natrium-, Magnesium- und Calcium-Ionen sind beispielsweise anionische Azofarbstoffe, Stilbenfarbstoffe und Merocyanine welche zumindest eine ionenselektive Gruppe (Ionophore Gruppe) enthalten und deren längstwellige Absorptionsbande sich zumindest teilweise mit der Emissionsbande des Luminophors überlappt, wobei die Wechselwirkung mit dem zu bestimmenden Kationen zu einer spektralen Verschiebung der längstwelligen Absorptionsbande führt.

Tabelle 2: Luminophore (Donorfarbstoffe)

| Luminophor (L) | Abkürzung | Absorptions-maximum (nm) | Lumineszenz-maximum (nm) |
|---|---|---|---|
| $(Ru(II)\text{-tris-}2,2'\text{-bibyridyl})^{2+}$ | $Ru(bpy)_3^{2+}$ | 452 | 628 |
| $(Ru(11)\text{-tris-}2,2'\ 4,4\text{-diphenyl bipyridyl})^{2+}$ | $Ru(dph\text{-}bpy)_3^{2+}$ | 474 | 632 |
| $(Ru(II)\text{-tris-}1,10\text{-phenanthrolin})^{2+}$ | $Ru(phen)_3^{2+}$ | 447 | 604 |
| | | | |
| $(Os(II)\text{-bis-terpyridin})^{2+}$ | | 510 | 729 |
| $(Os(II)\text{-tris-}1,10\text{-phenanthrolin})^{2+}$ | | 650 | 690 |

Als Zentralatome finden auch Ir, Rh, Pd, Pt oder Re Verwendung.

Die Erfindung wird im folgenden anhand der Abbildung 1 näher erläutert, wobei die Abbildung 1 eine Kalibrationskurve eines erfindungsgemäßen Reagens für die pH-Bestimmung zeigt.

Bestimmung der Kalibrierwerte

Die Bestimmung der Kalibrierkurven (Abbildung 1) und 02-Abhängigkeiten erfolgte durch die Bestimmung der Phasenverschiebung der Lumineszenz bezüglich des sinusförmig modulierten Anregungslichtes. Da ein langlebiger Luminophor verwendet wird, genügt eine einfache Meßanordnung mit einer Modulationsfrequenz von 45 kHz.
Zur Messung wurden Sensorscheiben aus den Sensorfolien herausgestanzt, diese am Ende eines 2-armigen Lichtleiterbündels befestigt und mit den jeweiligen Meßmedien in Kontakt gebracht bzw. das Ende des Lichtleiterbündels direkt in das, die Probe enthaltende Messmedium getaucht. Als Anregungslichtquelle wurde eine blaue LED (470 nm) verwendet, die mit einer auf 45 kHz sinusoidal modulierten Spannung der Amplitude 5 V versorgt wurde. Als Filter für das Anregungslicht wurden blaue Folienfilter verwendet. Das Anregungslicht wurde mittels Lichtleiter zur Sensorfolie bzw. Messflüssigkeit geführt. Das emittierte Lumineszenlicht wurde mittels Lichtleiterbündel zu einem Filter, eine Kombination aus einem OG 570 Glas Filter (Schott) und einem roten Folienfilter, geführt und weiters auf einen Detektor (Photomultiplier, Type Hamamatsu H5702) geleitet. Die sinusförmig modulierte Versorgungsspannung der LED und das Signal des Detektors wurde mittels eines Lock-In Verstärkers ausgewertet. Als Meßssignal wurde die Phasenverschiebung $\Phi$ erhalten.Die Abklingzeit $\tau$ wurde aus der gemessenen Phasenverschiebung $\Phi$ und der Modulationsfrequenz f = 45 kHz mit folgender Formel berechnet: $\tau = \tan(\Phi)/ (2 \pi f)$.

Als Meß- bzw. Kalibriermedien für das pH-Reagens (Abb. 1) wurden Phosphatpuffer verwendet, welche mit NaOH bzw. HCl auf die gewünschten pH-Werte eingestellt wurden. Die Puffer wurden durch Tonometrie mit Luft auf den Luftsauerstoffwert eingestellt bzw. durch Zugabe von $Na_2SO_3$ sauerstoffrei gemacht.

Abb. 1 zeigt die Kalibrationskurve eines auf dem FRET-Prinzip beruhenden optischen Reagens (hergestellt gemäß Abschnitt 2.3 des Beispielsteils) mit pH-sensitivem Akzeptorfarbstoff, wobei der Donorfarbstoff "geschützt" in einer erfindungsgemäßen Donorphase (Partikel) vorliegt. Die beiden Kurven zeigen den Phasenwinkel (Ordinate) des Lumineszenzlichts des in Kalibrationsflüssigkeiten unterschiedlicher pH-Werte (Abszisse) dispergierten pH-Reagens. Die mit "O" bezeichnete Kurve wurde mit Luft (21,95% $O_2$) gesättigt aufgenommen. Die mit "N" gekennzeichnete Kurve wurde mit $N_2$ gesättigt aufgenommen.

BEISPIELE

$Ru(diphphen)_3TMS_2$

[Ru(II)-tris-(4,7-diphenyl-1,10-phenanthroline) (3-trimethylsilyl)-1-propane sulphonate] (I. Klimant und O.S. Wolfbeis, Anal. Chem. 67 (1995) 3160).

Beispiel 1

Mit diesem Beispiel wird dokumentiert, daß ein Donorfarbstoff, welcher erfindungsgemäß in einer Donorphase eingebaut ist, weniger 02 Empfindlichkeit aufweist als derselbe Farbstoff, eingebaut in der Akzeptorphase.

Allgemeine Beschreibung der Herstellung von Nanopartikeln mit eingebautem Donorfarbstoff.

1.1 Einbetten eines Donorfarbstoffs in eine Akzeptorphase

1 mg des Donorfarbstoffs $Ru(diphphen)_3TMS_2$ und 100 mg des hydrophilen Polymers D4 (Polyurethan mit hydrophilen Sequenzen; Tyndale Plains Hunter LTD, Ringoes, NJ 08551, USA) wurden in Ethanol:Wasser (90:10 w/w) gelöst. Die Lösung wurde durch Rakeln auf eine Polyesterfolie (Mylar, Dupont) aufgezogen. Nach Abdunsten des Lösungsmittels entstand ein Film mit einer Schichtdicke von ca. 20 $\mu$m.

| Messanordnung (45 kHz, blaue LED, OG 570) | | | | |
|---|---|---|---|---|
| Trocken: | Luft | 55.4° | N2: | 58,3° |
| Wasser: | Luft gesättigt: | 53.5° | N2 gesättigt: | 58.4° |

Das Meßergebnis zeigt, daß der "ungeschützt" in einer Akzeptorphase vorliegende Donorfarbstoff $Ru(diphphen)_3TMS_2$, in Kontakt mit 02-freien (N2-Sättigung) bzw. 21.95% 02 (Luft) gesättigten gasförmigen und wässrigen Medien, eine 02-Empfindlichkeit von 2.9° (trockene Akzeptorphase) bzw. 4.9° (nasse Akzeptorphase) aufweist.

1.2 Herstellung der Nanopartikel mit Donorfarbstoff

400 mg Polyacrylnitril (Polyscience) und 8 mg Ru(diphphen)$_3$TMS$_2$ wurden in 80 ml DMF (Merck) gelöst. Nach langsamem Zutropfen von 500 ml Wasser wurde die entstandene Suspension mit NaCl versetzt und zentrifugiert. Das Zentrifugat wurde mehrmals mit Wasser und anschließend mit Ethanol gewaschen.

1.3 Einbetten der Donorfarbstofftragenden Nanopartikel in eine schichtförmige Akzeptorphase.

Die ethanolische Suspension (aus 1.2) wurde mit einer Lösung von 400 mg des hydrophilen Polymers D4 (Tyndale Plains Hunter LTD, Ringoes, NJ 08551, USA) in 5 ml Ethanol:Wasser (90:10, w:w) versetzt. Die Suspension wurde durch Rakeln auf eine Polyesterfolie (Mylar, Dupont) aufgezogen. Nach Abdunsten des Lösungsmittels entstand ein Film einer Schichtdicke von ca. 20 µm.

| Messanordnung (45 kHz, blaue LED, OG 570) | | | | |
|---|---|---|---|---|
| Trocken: | Luft | 57,4°; | N2: | 58,2° |
| Wasser: | Luftgesättigt: | 56.8° | O2-frei ($SO_3^{2-}$): | 58.6° |

Das Meßergebnis zeigt, daß der in ein erfindungsgemäßes Nanopartikel eingebaute, und somit "geschützt" in der Donorphase vorliegende Donorfarbstoff Ru(diphphen)$_3$TMS$_2$, in Kontakt mit 02-freien bzw. 21.95% 02 gesättigten, gasförmigen bzw. wässrigen Medien, eine 02-Empfindlichkeit von 0.8° (trockene Akzeptorphase) bzw. 1.6° (nasse Akzeptorphase) aufweist.

Durch Vergleich dieser Werte mit den Werten aus 1.1 wird somit ersichtlich, daß ein und derselbe in einer erfindungsgemäßen Donorphase vorliegende Donorfarbstoff weniger 02 Empfindlichkeit aufweist als der nach dem Stand der Technik direkt in der Akzeptorphase vorliegende Donorfarbstoff.

Beispiel 2 (erfindungsgemäßes Reagens)

Allgemeine Beschreibung der Herstellung pH-funktioneller Nanopartikel und einer pH-sensitiven Schicht.

In einem ersten Schritt wird ein OH-funktionelles Co-Polymer aus Acrylonitril und einem OH-funktionellen Methacrylat hergestellt. In einem zweiten Schritt werden aus diesem Polymer Nanopartikel mit eingebautem Donorfarbstoff hergestellt. In einem dritten Schritt wird ein pH-sensitiver Akzeptorfarstoff kovalent an die funktionellen Gruppen des Co-Polymers gebunden. Der Akzeptorfarbstoff ist dabei überwiegend in den "weichen" hydrophilen Bereichen (Akzeptorphase) der Partikel lokalisiert, und somit für Ionen zugänglich. Der Donorfarbstoff ist überwiegend in den "harten" Bereichen (Donorphase) gelöst und damit für störende Substanzen erschwert zugänglich.

2.1 Herstellung des Co-Polymers

230 g entionisiertes $H_2O$ wurden durch 2 h Spülen mit Stickstoff sauerstoffrei gemacht. Unter Rühren und Stickstoffatmosphäre wurden 4g SDS (Natriumdodecylsulfat p. A., Merck) gelöst. Zu dieser Lösung wurden 20 ml Acroylnitril (Fluka) und 1 ml Polyethylenglycolmonometacrylat (Polyscience, n=200, Bestell Nr. 16712) gegeben. Die Mischung wurde auf 50° gebracht und mit 4 ml 1 N HCl (Merck) versetzt. Die Polymerisierung wurde durch Zugabe von 400 mg Ammoniumperoxodisulfat (Merck) gestartet und 12 h bei 50° durchgeführt. Das Polymer wurde abgesaugt und mehrmals mit Wasser und Ethanol gewaschen. Dieses Polymer wird im folgenden als PAN-PEG bezeichnet.

2.2 Herstellung der Nanopartikel mit Donorfarbstoff

400 mg PAN-PEG und 20 mg des Donorfarbstoffs Ru(diphphen)$_3$TMS$_2$ wurden in 80 ml DMF (Merck) gelöst. Nach langsamen Zutropfen von 500 ml Wasser wurde die Suspension mit NaCl versetzt und zentrifugiert. Das Zentrifugat wurde mehrmals mit Wasser gewaschen.

2.3 Binden des pH-sensitiven Akzeptorfarbstoffes Chromophors an die Nanopartikel

25 mg des pH-sensitiven Akzeptorfarbstoffs N9 (Merck) wurden mit 8 Tr. $H_2SO_4$ konz. (Merck) verrieben und 30 min im Wasserstrahlvakkum aktiviert. Der Farbstoff wurde in 100 ml entionisiertem Wasser aufgenommen und mit NaOH auf pH 7 gebracht. Zu dieser Mischung wurde das oben beschriebene Zentrifugat gegeben, nach 5 min 4.2 g $NaCO_3$, nach 5 min 2 ml 5 M NaOH zugegeben. Nach weiteren 20 min wurde mit HCL konz. auf pH 3 angesäuert. Die Partikel wurden durch Zentrifugation abgetrennt und mit basischen Puffer, Wasser und Ethanol gewaschen.

Die so hergestellten Partikel wurden in ein wässriges Meßmedium suspendiert und die Messungen bei verschiedenen pH-Werten unter Luft- und N2-Sättigung vorgenommen. Das Ergebnis ist in der Abbildung 1 dargestellt und zeigt, daß die pH-sensitiven Partikel als Reagens (=Sonde bzw. engl. "Probe") zwecks pH-Bestimmung dem Meßmedium zugesetzt werden können.

Meßmedium: pH 7.3 Luftgesättigt: 50.0° O2-frei (N2 gesättigt): 51.3°

## Patentansprüche

1. Reagens zur Durchführung von Analysen nach dem FRET-Prinzip, enthaltend einen Akzeptorfarbstoff sowie Partikel, in die ein Donorfarbstoff eingebaut ist, wobei sich Donorfarbstoff und Akzeptorfarbstoff in verschiedenen chemischen Phasen befinden, der Akzeptorfarbstoff an die Partikel gebunden vorliegt und die Partikel für das Probenmedium oder für die Lumineszenzeigenschaften des Donorfarbstoffes beeinflussende Komponenten des Probenmediums impermeabel sind.

2. Reagens gemäß Anspruch 1, **dadurch gekennzeichnet, daß** als Material für die Partikel nicht weichgemachte Polymere eingesetzt werden.

3. Reagens gemäß Anspruch 2, **dadurch gekennzeichnet, daß** als Polymer Polyacrylnitril und Derivate davon, PVC und/oder Polyvinylidenchlorid eingesetzt wird.

4. Verfahren zur qualitativen und/oder quantitativen Bestimmung zumindest eines Analyten bzw. einer Komponente eines flüssigen Meßmediums, umfassend die Zugabe eines Reagens gemäß einem der Ansprüche 1 bis 3 zum Meßmedium.

5. Verfahren gemäß Anspruch 4, **dadurch gekennzeichnet, daß** es zur Bestimmung des pH-Wertes einer Probe oder zur Bestimmung der Konzentration bzw, der Aktivität von Ionen in einer Probe durchgeführt wird.

6. Verfahren gemäß Anspruch 5, **dadurch gekennzeichnet, daß** es zur Bestimmung der Konzentration bzw. Aktivität von $Li^+$, $Na^+$, $K^+$, $Mg^{++}$, $Ca^{++}$ oder $Cl^-$ durchgeführt wird.

7. Verfahren gemäß einem der Ansprüche 4 bis 6, **dadurch gekennzeichnet, daß** das Meßmedium eine Probe einer Körperflüssigkeit, insbesondere Blut, Plasma oder Serum ist

## Claims

1. A reagent for carrying out assays in accordance with the FRET-principle, containing an acceptor dye as well as particles in which a donor dye is incorporated, whereby the donor dye and the acceptor dye are located in different chemical phases, the acceptor dye is bound to the particles and the particles are impermeable to the sample medium or to components of the sample medium affecting the luminescence characteristics of the donor dye.

2. A reagent according to claim 1, **characterized in that** unplasticized polymers are used as materials for the particles.

3. A reagent according to claim 2, **characterized in that** polyacryl nitrile and derivatives thereof, PVC and/or polyvinylidene chloride are used as the polymer.

4. A method for the qualitative and/or quantitative determination of at least one analyte and/or component of a liquid measuring medium, comprising the addition of a reagent according to any of claims 1 to 3 to the measuring medium.

5. A method according to claim 4, **characterized in that** it is carried out for the determination of the pH-value of a sample or for the determination of concentrations and/or activities of ions in a sample.

6. A method according to claim 5, **characterized in that** it is carried out for the determination of concentrations and/or activities of $Li^+$, $Na^+$, $K^+$, $Mg^{++}$, $Ca^{++}$ or $Cl^-$.

**7.** A method according to any of claims 4 to 6, **characterized in that** the measuring medium is a sample of a body fluid, in particular blood, plasma or serum.

## Revendications

**1.** Réactif pour la réalisation d'analyses selon le principe FRET, contenant un colorant accepteur ainsi que des particules dans lesquelles est incorporé un colorant donneur, le colorant donneur et le colorant accepteur se trouvant dans des phases chimiques différentes, le colorant accepteur étant lié aux particules et les particules étant imperméables au milieu d'échantillonnage ou aux composants du milieu d'échantillonnage modifiant les propriétés de luminescence du colorant donneur.

**2.** Réactif selon la revendication 1, **caractérisé en ce que** l'on utilise, en tant que matériau pour les particules, des polymères non plastifiés.

**3.** Réactif selon la revendication 2, **caractérisé en ce que** l'on utilise, en tant que polymère, du polyacrylonitrile et ses dérivés, du PVC et/ou du poly(chlorure de vinylidène).

**4.** Procédé pour la détermination qualitative et/ou quantitative d'au moins d'un analyte ou d'un composant d'un milieu de mesure liquide, comprenant l'addition d'un réactif selon l'une des revendications 1 à 3 au milieu de mesure.

**5.** Procédé selon la revendication 4, **caractérisé en ce qu'**il est réalisé pour la détermination de la valeur du pH d'un échantillon ou pour la détermination de la concentration ou de l'activité des ions dans un échantillon.

**6.** Procédé selon la revendication 5, **caractérisé en ce qu'**il est effectué pour la détermination de la concentration ou de l'activité de $Li^+$, $Na^+$, $K^+$, $Mg^{++}$, $Ca^{++}$ ou $Cl^-$.

**7.** Procédé selon l'une des revendications 4 à 6, **caractérisé en ce que** le milieu de mesure est un échantillon de liquide corporel, en particulier du sang, du plasma ou du sérum.

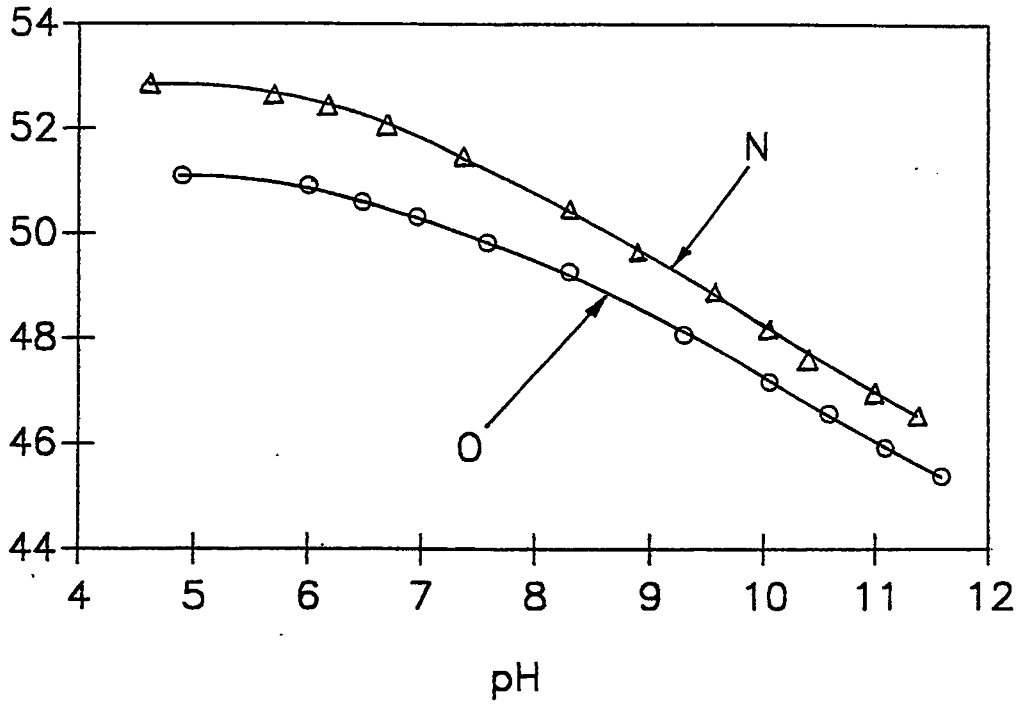


FIG. 1